# EUROPEAN PATENT SPECIFICATION

(11) **EP 4 442 674 B1**
(45) Date of publication and mention of the grant of the patent: **26.08.2026**
(21) Application number: 22901654.8
(22) Date of filing: 23.11.2022
(51) Int. Cl.: C07C 68/06, C07C 69/96, C07C 68/08

(54) **METHOD FOR PREPARING CARBONATE**
VERFAHREN ZUR HERSTELLUNG VON CARBONAT
PROCÉDÉ DE PRÉPARATION DE CARBONATE

(30) Priority: 30.11.2021 KR 20210167926
(43) Date of publication of application: 09.10.2024
(73) Proprietor: LOTTE CHEMICAL CORPORATION, Seoul 05551 (KR)
(72) Inventor: KIM, Jin Hyung, Daejeon 34110 (KR); BAEK, Mi Hwa, Daejeon 34110 (KR); KIM, Wang Gyu, Daejeon 34110 (KR); JEON, Hyo Jin, Daejeon 34110 (KR); HAN, Eunhye, Daejeon 34110 (KR)
(74) Representative: Germain Maureau
(86) International application number: PCT/KR2022/018579
(87) International publication number: WO 2023/101296

(56) References cited:
- CN-A- 1 320 594
- CN-A- 113 061 087
- CN-A- 113 582 845
- CN-A- 113 582 845
- CN-A- 113 636 935
- CN-A- 113 636 935
- CN-B- 109 776 322
- KR-A- 20150 055 022

## Description

### [Technical Field]

The present invention relates to a method for preparing a carbonate. Particularly, the present invention relates to a method capable of purifying a target product with high purity from a product of a carbonate preparation reaction.

### [Background Art]

As an organic solvent for a battery electrolyte, ethyl methyl carbonate (EMC) and diethyl carbonate (DEC) are mainly used. The EMC and DEC are prepared by transesterification of dimethyl carbonate (DMC) and ethanol (EtOH). The transesterification is also a reversible reaction. In addition, the transesterification may also be performed in the presence of a catalyst. Reaction Formula 1 below shows the transesterification (in which, MeOH is an abbreviation for methanol):

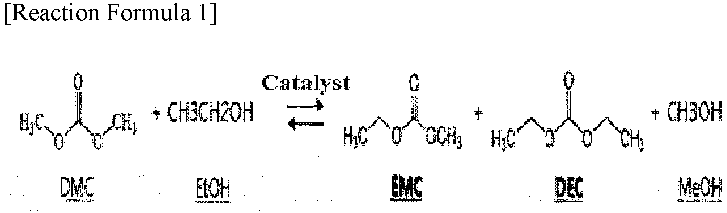

A catalyst applied to the reaction includes basic catalysts (NaOH, KOH, etc.) with excellent activity. However, the basic catalysts have low solubility in organic solvents, especially carbonates, and thus are not dissolved in DMC, EMC and DEC. Therefore, the basic catalysts cause column plugging, which is the cause of process defects in a reactive distillation process or purification process. That is, usually, the catalyst applied to the reaction is removed and then post-processing (e.g., separation and purification, etc.) is performed to produce a carbonate. The problem mainly occurs in a fluidized bed reactor.

Patent Document 1 discloses a method for preparing a carbonate using DMC, alcohol, and a sodium methoxide catalyst. In Patent Document 1, EMC and DEC are prepared through reactive distillation. Here, Patent Document 1 is intended to efficiently remove the catalyst by installing a strainer for separating solids (catalysts) at the rear end of a distillation column where reactive distillation is performed. However, when alcohol is distilled in the reactive distillation column, the catalyst is precipitated in the process. Accordingly, plugging occurs in the column because the precipitated catalyst accumulates inside the distillation column. In addition, Patent Document 1 uses two additional reactive distillation columns (second and third reaction distillation columns) except for a distillation column (first reactive distillation column) used for reaction distillation in a process of recovering the products EMC and DEC. Here, DEC is obtained from the bottom of the second reactive distillation column, and EMC is obtained from the bottom of the third reactive distillation column. When the product is obtained from the bottom of the distillation column, the product contains metals and impurities generated during the process, thereby making it difficult to obtain a high-purity carbonate. When the carbonate is applied to lithium-ion batteries, the battery resistance increases. In addition, in order to first obtain DEC (boiling point: 125.8°C), which has a higher boiling point than EMC (boiling point: 107°C), a higher heat (transfer) load and heat transfer rate are required in a heat exchanger after the reaction. Therefore, according to the method of Patent Document 1, energy costs increase.

Patent Document 2 discloses a method for preparing a carbonate through extractive distillation by using a gel-type strong basic ion exchange resin catalyst as a transesterification catalyst. The extractive distillation requires a separation process of separating the product with an extractant, which increases equipment and energy costs. In addition, since an ion exchange resin catalyst usually has a low strength, the deactivation progresses quickly due to a loss of exchange groups and formation of fine powder. As a result, since the pressure in the reactor also increases, the catalyst needs to be replaced periodically. In addition, since a trace of extractant may be present in the carbonate, additional processes are required to increase the purity.

Therefore, research is required on methods capable of effectively preparing high-purity EMC and DEC with a high DMC conversion rate.

### [Prior Arts]

### [Patent Documents]

(Patent Document 1) CN 103804124 B
(Patent Document 2) KR 10-2011-0105379 A

### [Disclosure]

### [Technical Problem]

An object of the present invention is to effectively prepare high-purity EMC and DEC with a high DMC conversion rate.

Another object of the present invention is to reduce energy costs and maintenance costs by reducing a load on a heat exchanger.

### [Technical Solution]

An aspect of the present invention provides a method for preparing a carbonate including a first step of preparing a product containing ethyl methyl carbonate, diethyl carbonate, and methanol by adding raw materials containing dimethyl carbonate and ethanol into a fixed bed reactor system and inducing transesterification of the raw materials; a second step of separating a reactor effluent containing the product and the unreacted raw materials from a first separation system to obtain a first separation system overhead stream including ethanol, methanol and dimethyl carbonate as main components and a first separation system bottom stream including diethyl carbonate and ethyl methyl carbonate as main components; a third step of separating the first separation system bottom stream from a second separation system to obtain a second separation system overhead stream including ethyl methyl carbonate as a main component, and a second separation system bottom stream including diethyl carbonate as a main component; and a fourth step of separating the second separation system bottom stream from a third separation system to obtain a third separation system overhead stream including diethyl carbonate as a main component, in which any one of the second to fourth steps sets a reflux ratio of any one of the first to third separation systems in the range of 0.5 to 5.

### [Advantageous Effects]

According to the method for preparing the carbonate of the present invention, it is possible to effectively prepare high-purity EMC and DEC with a high DMC conversion rate.

Further, it is possible to reduce energy costs and maintenance costs by reducing a load on a heat exchanger.

### [Description of Drawings]

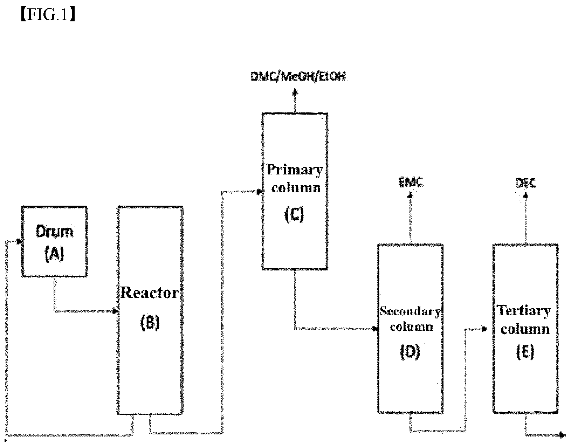

FIG. 1 is a schematic diagram of a method for preparing a carbonate according to the present invention.

### [Best Mode of the Invention]

Hereinafter, the contents of the present invention will be described in more detail.

The present invention relates to a method for preparing a carbonate. Specifically, the present invention relates to a method for preparing a liquid carbonate or a carbonate-based solvent. The present invention is intended to obtain a carbonate with high purity. In addition, the present invention is intended to easily perform a process of obtaining the carbonate with high energy efficiency and low costs.

The present invention is intended to prepare a carbonate obtained by transesterification of DMC and ethanol. To this end, in the present invention, largely, transesterification of DMC and ethanol is performed, and then a process of purifying the reaction product is performed.

To this end, the method of the present invention proceeds through at least four steps.

In the method of the present invention, in a first step, a product containing ethyl methyl carbonate, diethyl carbonate, and methanol is prepared by adding raw materials containing dimethyl carbonate and ethanol into a fixed bed reactor system, and inducing transesterification of the raw materials.

The product containing ethyl methyl carbonate, diethyl carbonate, and methanol may be prepared through transesterification of dimethyl carbonate and ethanol.

The reactors using the catalyst are broadly divided into two types. The first type is a fluidized bed reactor, and the second type is a fixed bed reactor.

In the fluidized bed reactor, literally, the catalyst flows in the reactor. For this reason, since the catalyst may be included in the product, it is required to remove the catalyst before the process of separating or purifying the product. This is because when the catalyst is not removed, column plugging occurs, which is the cause of defects in the separation and/or purification process.

In the fixed bed reactor, the catalyst is present in the fixed state in the reactor. Accordingly, even when the reaction is completed, the catalyst is still present in the reactor, so that the product does not contain the catalyst. Therefore, when using the fixed bed reactor, a separate process of removing the catalyst is not required. In the present invention, the fixed bed reactor is applied. As a result, the method of the present invention does not require a process of removing the catalyst applied to the reaction, and there is no risk of the above-mentioned problems occurring due to the catalyst.

In the present invention, the raw materials prepared above are added into the reactor system. In this specification, the term "reactor system" is used as a concept of encompassing other facilities attached to the reactor, or other additional reactors, not the reactor alone. That is, the reactor system includes a single reactor and other facilities attached thereto, a plurality of reactors, or a plurality of reactors and other facilities attached thereto.

In the method of the present invention, the transesterification of dimethyl carbonate and ethanol in the raw materials is performed in the reactor system. As a result, the product including ethyl methyl carbonate (EMC), diethyl carbonate (DEC), and methanol (MeOH) is prepared. This product is discharged from the reactor system as a reactor effluent, together with unreacted raw materials. As described above, since the method of the present invention uses the fixed bed reactor, the reactor effluent may not contain catalyst.

In the method of the present invention, in a second step, the reactor effluent containing the product and the unreacted raw materials is separated. Specifically, in the method of the present invention, the reactor effluent containing the product and the unreacted raw materials is separated from a first separation system to obtain a first separation system overhead stream including ethanol, methanol and dimethyl carbonate as main components and a first separation system bottom stream including diethyl carbonate and ethyl methyl carbonate as main components. More specifically, in the method of the present invention, in the second step, the reactor effluent is supplied to the first separation system, and the first separation system discharges low boiling-point components of the reactor effluent from the column overhead of the first separation system, and discharges high boiling-point components of the reactor effluent from the column bottom of the first separation system.

In this specification, a stream discharged from the column overhead of the separation system is referred to as a separation system overhead stream.

In this specification, a stream discharged from the column bottom of the separation system is referred to as a separation system bottom stream.

Among the components included in the reactor effluent, the low-boiling point components include ethanol, methanol, and dimethyl carbonate as main components. In addition, among the components included in the reactor effluent, the high-boiling point components include diethyl carbonate and ethyl methyl carbonate as main components.

In this specification, the including of a specific component as a main component may mean that the corresponding component is included in a ratio of 60 wt% or more, 65 wt% or more, 70 wt% or more, 75 wt% or more, 80 wt% or more, 85 wt% or more, 90 wt% or more, 95 wt% or more, 97 wt% or more, or 99 wt% or more based on the total amount, and included in a ratio of less than 100 wt%, or approximately 100 wt%.

In addition, in this specification, the separation system is used as a concept of encompassing not only a separation column, which is a column-shaped structure that performs a separation process, but also a reboiler for reheating a part of the stream refluxed to the separation column in the bottom stream thereof, and a cooler for cooling a part of the stream refluxed to the separation column (or a condenser for condensing the stream thereof) in the overhead stream thereof.

In the method of the present invention, in a third step, the first separation system bottom stream is separated. Specifically, in the method of the present invention, in the third step, the first separation system bottom stream is separated from a second separation system to obtain a second separation system overhead stream including ethyl methyl carbonate as a main component and a second separation system bottom stream including diethyl carbonate as a main component. More specifically, in the method of the present invention, in the second step, the first separation system bottom stream is supplied to the second separation system and the first separation system bottom stream is separated from the second separation system to obtain a second separation system overhead stream including ethyl methyl carbonate as a main component and a second separation system bottom stream including diethyl carbonate as a main component.

In the method of the present invention, in a fourth step, the second separation system bottom stream is separated. Specifically, in the method of the present invention, in the fourth step, the second separation system bottom stream is separated from a third separation system to obtain a third separation system overhead stream containing diethyl carbonate as a main component. More specifically, in the method of the present invention, in the fourth step, the second separation system bottom stream is separated from a third separation system to obtain a third separation system overhead stream containing diethyl carbonate, which is a low boiling-point component as a main component and a third separation system bottom stream containing a residual heavier, which is a high boiling-point component as a main component. That is, in the present invention, in the fourth step, the second separation system bottom stream is supplied to the third separation system, and the second separation system bottom stream is separated from the third separation system to obtain a third separation system overhead stream containing diethyl carbonate as a main component.

The second to fourth steps are different from existing methods in which high boiling point components are first separated and discharged. In the steps, the low boiling point components are first separated and discharged. Through this, in the method of the present invention, it is possible to reduce a load on a heat exchanger required for separation, thereby reducing energy costs and maintenance costs.

In addition, in the present invention, a high-purity carbonate of 99.9% or more may be obtained only when the operating conditions of the separation system are appropriately adjusted. Specifically, in the method of the present invention, any one of the second to fourth steps sets a reflux ratio of any one of the first to third separation systems in the range of 0.5 to 5. That is, the reflux ratio of at least one of the separation systems applied in the present invention needs to be adjusted as above. In another embodiment, in at least two of the second to fourth steps, the reflux ratios of at least two of the first to third separation systems may be set in the range of 0.5 to 5.

Even if the same method as the present invention is applied, various problems occur when the reflux ratio of the entire separation system is out of the above range. Specifically, when the reflux ratio of the entire separation system is less than 0.5, a ratio of the amount of reflux liquid to the amount of effluent liquid at the column overhead is small, and thus the discharge amount of the product increases, but it is difficult to obtain a high-purity carbonate. In addition, if the reflux ratio of the entire separation system is more than 5, the ratio of the amount of reflux liquid to the amount of effluent liquid at the column overhead increases to obtain a high-purity carbonate. However, since the amount of effluent liquid is small, the product obtainment time increases and the operating cost increases.

In this specification, a reflux ratio (R) refers to a ratio of the amount of reflux liquid to the amount of effluent liquid. That is, based on the amount of liquid phase discharged from a specific column or reactor, the amount of liquid phase returning to the column or reactor may be referred to as the reflux ratio.

FIG. 1 briefly illustrates a process of performing the method of the present invention. The contents of FIG. 1 may be summarized as follows. A drum A is a container that stores a solution (sulfuric acid/NaOH, etc.) for reusing an ion exchange resin. The drum A may regenerate the ion exchange resin by circulating the solution when the efficiency of the ion exchange resin decreases. A reactor B is a reactor system. The reactor B operates with a plurality of fixed bed reactors connected in series. A bottom material discharged from the reactor is transmitted to a first separation system C. DMC/MeOH/EtOH is obtained at the top of the first separation system C and EMC/DEC is obtained at the bottom thereof. EMC is recovered from the top of a second separation system D. A bottom effluent is transmitted to a third separation system E. DEC is recovered from the top of the third separation system E, and a heavier is discarded from the bottom of the third separation system.

In an embodiment, a mixing ratio of reactants DMC and EtOH in the raw materials may also be appropriately adjusted. For example, a ratio (EtOH/DMC) of the weight of dimethyl carbonate (DMC) and the weight of ethanol (EtOH) in the raw materials may be in the range of 0.1 to 10. In another embodiment, the ratio (EtOH/DMC) may be 0.15 or higher or 0.2 or higher, and may be 9 or less, 8 or less, 7 or less, 6 or less, or 5 or less. Within the range, the reactants may properly contact the catalyst to perform the reaction, and waste of energy may be reduced in a subsequent purification process. In addition, the ratio between the reactants may also be adjusted depending on what a target product is. For example, since the demand for DEC is high, it is common to set the composition to increase its selectivity by using DEC as a target product, but it is expected that the demand for EMC is high in the future.

The reactor system may also be designed appropriately.

In an embodiment, the fixed bed reactor may be a fixed bed reactor filled with an ion exchange resin catalyst. It is possible to maximize a contact with reactants by applying the appropriate ion exchange resin catalyst. As a result, it is possible to prepare the target product, carbonate with a fast reaction rate and high reaction efficiency. In an embodiment, the ion exchange resin catalyst may be a basic ion exchange resin catalyst or an acidic ion exchange resin catalyst. Specifically, the ion exchange resin catalyst may be a basic ion exchange resin catalyst.

The acidic ion exchange resin may be divided into a strongly acidic ion exchange resin and a weakly acidic ion exchange resin. Usually, the strongly acidic ion exchange resin catalyst may be used, and in this case, the resin catalyst may have a sulfonate ion as an exchange group and a hydrogen ion as an ion type.

The basic ion exchange resin catalyst may be divided into a weakly basic ion exchange resin catalyst and a strongly basic ion exchange resin catalyst. The strongly basic ion exchange resin catalyst may have bromide anion (Br⁻), thiocyanate (SCN⁻) anion, chlorine anion (Cl⁻), acetate anion (acetate, CH₃COO⁻), hydroxyl anion (OH⁻), fluorine anion (F⁻), etc. as ionic types. The weakly basic ion exchange resin catalyst may have citrate anion (C₃H₅O(COO)₃³⁻), sulfate (SO₄²⁻), etc. as ionic types.

In an embodiment, the ion exchange resin catalyst may be a porous ion exchange resin catalyst or a gel-type ion exchange resin catalyst. Specifically, the ion exchange resin catalyst may be a porous ion exchange resin catalyst. A porous ion exchange resin has many (about 10 to 1,000) macropores on the surface to be chemically more stable than a gel-type resin. In addition, the porous ion exchange resin may have a large surface area to maximize a contact area with reactants.

The porous ion exchange resin may have excellent decolorization to relatively reduce the washing time of the ion exchange resin and the amount of washing solvent used at the early reaction. In addition, the porous ion exchange resin has high strength, and when reused, a loss of the exchange groups and formation of fine powder are small, so that the reuse cycle is long.

In an embodiment, the ion exchange resin catalyst may have an exchange capacity of 1 (eq/l-wet resin) or more and 1.5 (eq/l-wet resin) or less. In general, as the crosslinking degree of the ion exchange resin increases, the exchange capacity tends to be large because more exchange groups are attached to the parent matrix. However, when the crosslinking degree of the ion exchange resin is high, the number of exchange groups participating in the reaction decreases due to the strong bond between the parent matrix and the exchange groups, and thus the reaction efficiency decreases. Accordingly, the strength may be maintained even under an appropriate reaction rate and a rapid volume change within the exchange capacity range.

Therefore, it may be most advantageous to apply an ion exchange resin catalyst that satisfies all of the characteristics. In an embodiment, the fixed bed reactor system includes a fixed bed reactor filled with a basic ion exchange resin catalyst, the basic ion exchange resin is porous, and the exchange capacity of the basic ion exchange resin may be in the range of 1 (eq/l-wet resin) to 1.5 (eq/l-wet resin).

In addition, the present invention may appropriately design reactor facilities of the reactor system. In the present invention, the fixed bed reactor system may be a system having a plurality of fixed bed reactors. That is, the fixed bed reactor system may include two or more fixed bed reactors. In addition, in the present invention, the fixed bed reactor system may connect the plurality of fixed bed reactors in series. Here, the connecting of the plurality of reactors in series means that an outlet of one reactor is connected to an inlet of the other reactor. In an embodiment, the fixed bed reactor system may include a plurality of fixed bed reactors connected in series.

In the reactor system, the number of fixed bed reactors may be appropriately adjusted. The fixed bed reactor system may be a system in which 2 to 10 fixed bed reactors are connected in series.

Unlike existing methods, in the present invention, reactive distillation or extractive distillation may not be performed. That is, the method of the present invention may be applied with a tray column or packing column as the separation system. Since the meaning of the tray column and the packing column is known, the detailed descriptions will be omitted. Since theoretical and empirical data related to the design are rich, it possible to design a systematic separation and purification system by using the tray column and the packing column. That is, in an embodiment, the first to third separation systems may all be tray columns or packing columns.

When the previous process has corresponded to the reaction process in the method of the present invention, a process to be described below may be a separation (or purification) process. The operating conditions of the separation system applied during the separation or purification process using the separation system may also be appropriately adjusted.

The separation and purification conditions in the second step may also be appropriately adjusted.

In one example, the fourth step may be applied with a separation system with the theoretical number of stages in the range of 15 to 80 stages as the first separation system. Within the above range, a separation system may be manufactured economically at the same time while exhibiting appropriate stage efficiency. In addition, within the range, maintenance is easy and the heat balance of the separation system may be easily adjusted.

In one example, in the second step, the reflux ratio of the first separation system may be set in the range of 0.5 to 5. The effect of this setting is the same as described above.

In addition, the combination of the first and second steps has a mechanism similar to that of conventional reactive distillation. However, in the third and fourth steps, the target products are discharged in the order of boiling points through the column overhead, which is a clear difference from the conventional method of discharging the target products in the opposite order through the column bottom. In particular, in the present invention, to this end, it is required to adjust the operating conditions in each of the second to fourth steps.

In one example, in the second step, the temperature of the reboiler of the first separation system may be set in the range of 90°C to 100°C. At this time, the overhead stream of the first separation system may contain ethanol, methanol, and DMC as main components, and the bottom stream of the first separation system may contain DEC and EMC as main components.

The separation and purification conditions in the third step may also be appropriately adjusted.

In one example, the third step may be applied with a separation system with the theoretical number of stages in the range of 15 to 80 stages as the second separation system. Within the above range, the separation system may be manufactured economically at the same time while exhibiting appropriate stage efficiency. In addition, within the range, maintenance is easy and the heat balance of the separation system may be easily adjusted.

In one example, in the third step, the reflux ratio of the second separation system may be set in the range of 0.5 to 5. The effect of this setting is the same as described above.

In one example, in the third step, the temperature of the reboiler of the second separation system may be set in the range of 110°C to 120°C. At this time, the overhead stream of the second separation system may contain EMC as a main component, and the bottom stream of the second separation system may contain DEC as a main component.

The separation and purification conditions in the fourth step may also be appropriately adjusted.

In one example, the fourth step may be applied with a separation system with the theoretical number of stages in the range of 15 to 80 stages as the third separation system. Within the above range, the separation system may be manufactured economically at the same time while exhibiting appropriate stage efficiency. In addition, within the range, maintenance is easy and the heat balance of the separation system may be easily adjusted.

In one example, in the fourth step, the reflux ratio of the third separation system may be set in the range of 0.5 to 5. The effect of this setting is the same as described above.

In one example, in the fourth step, the temperature of the reboiler of the third separation system may be set in the range of 130°C to 140°C. At this time, the overhead stream of the third separation system may contain DEC as a main component, and the bottom stream of the third separation system may contain a residual heavier as a main component.

Meanwhile, the method for preparing the carbonate of the present invention may further include conventional steps known in the art before or after each of the steps, in addition to the above-described steps.

Hereinafter, the present invention will be described in more detail. However, the following Examples do not limit the content of the present invention.

### [Gas chromatography]

The analysis of the purity of a product and the contents of impurities and heavier in the product were conducted using gas chromatography (GC). Specifically, GC was performed on a sample in which 1 g of an analyte material was taken and mixed with 0.1 g of m-xylene. An YL6500GC product from YOUNGIN Chromas was used as the GC equipment, in which a GC column was DB-1 30 m * 0.32 mm from Agilent and a GC detector was FID.

### [ICP-AES]

The analysis of the metal content in the product was measured using inductively coupled plasma atomic emission spectroscopy (ICP-AES). An Optima 8300 model from Perkin Elmer was used. 10 g of an effluent sample was mixed with 10 ml of 70% nitric acid and 10 ml of water, heated at 250°C for 2 hours, and then analyzed.

### [Color]

Product colors were evaluated by APHA. The APHA is also called Hazen or Pt/Co color, which refers to the yellowness of a liquid sample. For APHA measurement, an automatic color meter from Tintometer, a PFX 995 model, was used. A sample was filled with about 3/4 of a cell height, disposed in close contact with a right position, and then analyzed by setting a temperature to room temperature and pressing a READ key. After measuring three times, an average value was taken as a result value.

### [Examples]

A reaction system was prepared in which four fixed bed reactors were connected in series. An ion exchange resin for each reactor was filled with 80 cc (58 g) of a highly basic porous anion exchange resin (Samyang Corporation, Trilte AMP-18), which had trimethylammonium (TMA) ions as an exchange group, chlorine ions as an ion type, and an exchange capacity of 1.3 eq/L. The raw materials included ethanol (EtOH) and DMC at a mass ratio (EtOH/DMC) of 0.8. The system was operated while continuously supplying raw materials to the reactor system at 2 cc/min and maintaining the temperature of the system at 70°C.

The reaction product was supplied to a first separation system. The number of stages of the first separation system was 60. Separation was performed at a reflux ratio of 3, normal pressure, and a reboiler temperature of 93 to 100°C. An effluent containing DMC, EtOH, and MeOH as main components was obtained from the column overhead, and an effluent containing EMC and DEC as main components was obtained from the column bottom.

The bottom effluent from the first separation system was supplied to a second separation system. The number of stages of the second separation system was 60. Separation was performed at a reflux ratio of 3, normal pressure, and a reboiler temperature of 110 to 120°C. From the column overhead of the second separation system, EMC with the purity of 99.9% or more and a total metal content of 1 ppm or less was obtained. An effluent containing DEC as a main component was obtained from the column bottom of the second separation system.

The bottom effluent of the second separation system was supplied to a third separation system. The number of stages of the third separation system was 60. Separation was performed at a reflux ratio of 3, normal pressure, and a reboiler temperature of 130 to 140°C. From the column overhead of the third separation system, DEC with the purity of 99.9 % or more and a total metal content of 1 ppm or less was obtained. The heavier obtained from the column bottom of the third separation system was discarded.

### [Comparative Example 1]

The same process as Example 1 was performed, except that the reflux ratios in the first to third separation systems were all set to 0.3.

### [Comparative Example 2]

The same process as Example 1 was performed, except that the third separation system was not applied and the operation was performed to obtain the bottom stream of the second separation system containing DEC as the main component. The results of Examples and Comparative Examples 1 and 2 were compared and shown in Tables 1 and 2 below.

**[Table 1]**

| Result | Purity | EMC [%] | DEC [%] |
|---|---|---|---|
| | Example | 99.99 | 99.99 |
| | Comparative Example 1 | 99.8 | 99.8 |

**[Table 2]**

| Result | Classification | Example | Comparative Example 2 |
|---|---|---|---|
| | DEC purity [%] | 99.99 | 99.5 |
| | Impurity/Heavier content [%] | - | 0.5 |
| | DEC total metal content [ppm] | 1 | 5 |
| | APHA (color) | 3 | 10 |

According to Table 1, it may be confirmed that it is required to satisfy the reflux ratio of the separation system according to the present invention. If the reflux ratio is less than 0.5, a ratio of the amount of reflux liquid to the amount of effluent liquid in the column overhead is small, and the discharge amount of the product increases. However, it is difficult to obtain a carbonate with high purity of 99.9% or higher.

According to Table 2, if the number of separation systems is not sufficiently used and DEC is obtained at the column bottom, it is difficult to obtain a high-purity carbonate. In this case, since there are many impurities and the APHA value is too high, it is difficult to use the carbonate as an organic solvent for lithium ion batteries. Therefore, considering the high purity, the metal content, and the APHA value, it may be seen that the carbonate should be prepared in the same manner as the method of the present invention.

## Claims

1. A method for preparing a carbonate comprising:
a first step of preparing a product containing ethyl methyl carbonate, diethyl carbonate, and methanol by adding raw materials containing dimethyl carbonate and ethanol into a fixed bed reactor system and inducing transesterification of the raw materials;
a second step of separating a reactor effluent containing the product and the unreacted raw materials from a first separation system to obtain a first separation system overhead stream including ethanol, methanol and dimethyl carbonate as main components and a first separation system bottom stream including diethyl carbonate and ethyl methyl carbonate as main components;
a third step of separating the first separation system bottom stream from a second separation system to obtain a second separation system overhead stream including ethyl methyl carbonate as a main component, and a second separation system bottom stream including diethyl carbonate as a main component; and
a fourth step of separating the second separation system bottom stream from a third separation system to obtain a third separation system overhead stream including diethyl carbonate as a main component,
wherein any one of the second to fourth steps sets a reflux ratio of any one of the first to third separation systems in the range of 0.5 to 5.

2. The method for preparing the carbonate of claim 1, wherein the fixed bed reactor system includes a fixed bed reactor filled with a basic ion exchange resin catalyst,
the basic ion exchange resin is porous, and
the exchange capacity of the basic ion exchange resin is in the range of 1 (eq/l-wet resin) to 1.5 (eq/l-wet resin).

3. The method for preparing the carbonate of claim 1, wherein the fixed bed reactor system includes a plurality of fixed bed reactors connected in series.

4. The method for preparing the carbonate of claim 1, wherein the first separation system to the third separation system are tray columns or packing columns.

5. The method for preparing the carbonate of claim 1, wherein in the second step, a separation system with the theoretical number of stages in the range of 15 to 80 stages is applied as the first separation system, the reflux ratio of the first separation system is set in the range of 0.5 to 5, and the temperature of a reboiler of the first separation system is set in the range of 90°C to 100°C.

6. The method for preparing the carbonate of claim 1, wherein in the third step, a separation system with the theoretical number of stages in the range of 15 to 80 stages is applied as the second separation system, the reflux ratio of the second separation system is set in the range of 0.5 to 5, and the temperature of a reboiler of the second separation system is set in the range of 110°C to 120°C.

7. The method for preparing the carbonate of claim 1, wherein in the fourth step, a separation system with the theoretical number of stages in the range of 15 to 80 stages is applied as the third separation system, the reflux ratio of the third separation system is set in the range of 0.5 to 5, and the temperature of a reboiler of the third separation system is set in the range of 130°C to 140°C.

## Patentansprüche

1. Verfahren zur Herstellung eines Karbonats, umfassend:
einen ersten Schritt des Vorbereitens eines Produkts, das Ethylmethylcarbonat, Diethylcarbonat und Methanol enthält, indem Dimethylcarbonat und Ethanol enthaltende Rohstoffe in ein Festbettreaktorsystem gegeben werden und die Transesterifizierung der Rohstoffe induziert wird;
einen zweiten Schritt des Trennens eines Reaktorabwassers, das das Produkt und die unreagierten Rohstoffe enthält, von einem ersten Trennsystem, um einen ersten Oberstrom des Trennsystems zu erhalten, der Ethanol, Methanol und Dimethylcarbonat als Hauptbestandteile einschließt und einen ersten Bodenstrom des Trennsystems, der Diethylcarbonat und Ethylmethylcarbonat als Hauptbestandteile einschließt;
einen dritten Schritt zum Trennen des ersten Trennsystem-Bodenstroms von einem zweiten Trennsystem, um einen zweiten Trennsystem-Oberstrom einschließlich Ethylmethylcarbonat als Hauptkomponente und einen zweiten Trennsystem-Bodenstrom einschließlich Diethylcarbonat als Hauptkomponente zu erhalten; und
einen vierten Schritt zum Trennen des zweiten Trennsystem-Bodenstroms von einem dritten Trennsystem, um einen dritten Trennsystem-Oberstrom zu erhalten, der Diethylcarbonat als Hauptkomponente einschließt,
wobei einer der zweiten bis vierten Schritte ein Rückflussverhältnis eines der ersten bis dritten Trennsysteme im Bereich von 0,5 bis 5 einstellt.

2. Verfahren zur Vorbereitung des Karbonats nach Anspruch 1, wobei das Festbettreaktorsystem einen mit einem basischen Ionenaustauscherharz-Katalysator gefüllten Festbettreaktor einschließt,
das basische Ionenaustauscherharz porös ist, und
die Austauschkapazität des basischen Ionenaustauschharzes im Bereich von 1 (eq/I-Nassharz) bis 1,5 (eq/I-Nassharz) liegt.

3. Verfahren zur Vorbereitung des Karbonats nach Anspruch 1, wobei das Festbettreaktorsystem eine Vielzahl von in Reihe geschalteten Festbettreaktoren umfasst.

4. Verfahren zur Herstellung des Karbonats nach Anspruch 1, wobei das erste Trennsystem zum dritten Trennsystem Traysäulen oder Packsäulen sind.

5. Verfahren zur Herstellung des Karbonats nach Anspruch 1, wobei im zweiten Schritt ein Trennsystem mit der theoretischen Anzahl von Stufen im Bereich von 15 bis 80 Stufen als erstes Trennsystem angewendet wird, das Rückflussverhältnis des ersten Trennsystems im Bereich von 0,5 bis 5 eingestellt ist und die Temperatur eines Umkessels des ersten Trennsystems im Bereich von 90 °C bis 100 °C eingestellt ist.

6. Verfahren zur Herstellung des Karbonats nach Anspruch 1, wobei im dritten Schritt ein Trennsystem mit der theoretischen Anzahl von Stufen im Bereich von 15 bis 80 Stufen als zweites Trennsystem angewendet wird, das Rückflussverhältnis des zweiten Trennsystems im Bereich von 0,5 bis 5 eingestellt ist und die Temperatur eines Umkessels des zweiten Trennsystems im Bereich von 110 °C bis 120 °C eingestellt ist.

7. Verfahren zur Herstellung des Karbonats nach Anspruch 1, wobei im vierten Schritt ein Trennsystem mit der theoretischen Anzahl von Stufen im Bereich von 15 bis 80 Stufen als drittes Trennsystem angewendet wird, das Rückflussverhältnis des dritten Trennsystems im Bereich von 0,5 bis 5 eingestellt ist und die Temperatur eines Umkessels des dritten Trennsystems im Bereich von 130 °C bis 140 °C eingestellt ist.

## Revendications

1. Procédé de préparation de carbonate comprenant :
une première étape de préparation d'un produit contenant du carbonate d'éthyle et de méthyle, du carbonate de diéthyle et du méthanol en ajoutant des matières premières contenant du carbonate de diméthyle et de l'éthanol dans un système de réacteur à lit fixe et en provoquant la transestérification des matières premières ;
une deuxième étape de séparation d'un effluent de réacteur contenant le produit et les matières premières n'ayant pas réagi d'un premier système de séparation pour obtenir un flux de tête du premier système de séparation comprenant de l'éthanol, du méthanol et du carbonate de diméthyle en tant que composants principaux et un flux de fond du premier système de séparation comprenant du carbonate de diéthyle et du carbonate d'éthyle et de méthyle en tant que composants principaux ;
une troisième étape de séparation du flux de fond du premier système de séparation d'un deuxième système de séparation pour obtenir un flux de tête du deuxième système de séparation comprenant du carbonate d'éthyle et de méthyle en tant que composant principal, et un flux de fond du deuxième système de séparation comprenant du carbonate de diéthyle en tant que composant principal ; et
une quatrième étape de séparation du flux de fond du deuxième système de séparation d'un troisième système de séparation pour obtenir un flux de tête du troisième système de séparation comprenant du carbonate de diéthyle en tant que composant principal,
dans lequel l'une quelconque des deuxième à quatrième étapes définit un rapport de reflux de l'un quelconque des premier à troisième systèmes de séparation dans la plage allant de 0,5 à 5.

2. Procédé de préparation du carbonate selon la revendication 1, dans lequel le système de réacteur à lit fixe comprend un réacteur à lit fixe rempli d'un catalyseur à résine échangeuse d'ions basique,
la résine échangeuse d'ions basique est poreuse, et
la capacité d'échange de la résine échangeuse d'ions basique se trouve dans la plage allant de 1 (eq/l de résine humide) à 1,5 (eq/l de résine humide).

3. Procédé de préparation du carbonate selon la revendication 1, dans lequel le système de réacteur à lit fixe comprend une pluralité de réacteurs à lit fixe reliés en série.

4. Procédé de préparation du carbonate selon la revendication 1, dans lequel le premier système de séparation jusqu'au troisième système de séparation sont des colonnes à plateaux ou des colonnes à garnissage.

5. Procédé de préparation du carbonate selon la revendication 1, dans lequel, lors de la deuxième étape, un système de séparation ayant un nombre théorique d'étages dans la plage allant de 15 à 80 étages est appliqué comme étant le premier système de séparation, le rapport de reflux du premier système de séparation est défini dans la plage allant de 0,5 à 5, et la température d'un rebouilleur du premier système de séparation est définie dans la plage allant de 90°C à 100°C.

6. Procédé de préparation du carbonate selon la revendication 1, dans lequel, lors de la troisième étape, un système de séparation ayant un nombre théorique d'étages dans la plage allant de 15 à 80 étages est appliqué comme étant le deuxième système de séparation, le rapport de reflux du deuxième système de séparation est défini dans la plage allant de 0,5 à 5, et la température d'un rebouilleur du deuxième système de séparation est définie dans la plage allant de 110°C à 120°C.

7. Procédé de préparation du carbonate selon la revendication 1, dans lequel, lors de la quatrième étape, un système de séparation ayant un nombre théorique d'étages dans la plage allant de 15 à 80 étages est appliqué comme étant le troisième système de séparation, le rapport de reflux du troisième système de séparation est défini dans la plage allant de 0,5 à 5, et la température d'un rebouilleur du troisième système de séparation est définie dans la plage allant de 130°C à 140°C.
